Europäisches Patentamt

(19) European Patent Office

Office européen des brevets

(11) **EP 0 951 351 B1**

(12) **EUROPÄISCHE PATENTSCHRIFT**

(45) Veröffentlichungstag und Bekanntmachung des Hinweises auf die Patenterteilung:
**09.05.2001 Patentblatt 2001/19**

(51) Int Cl.$^7$: **B01J 23/22**, B01J 37/02, C07C 51/25

(21) Anmeldenummer: **97951986.5**

(22) Anmeldetag: **27.11.1997**

(86) Internationale Anmeldenummer:
**PCT/EP97/06612**

(87) Internationale Veröffentlichungsnummer:
**WO 98/23371 (04.06.1998 Gazette 1998/22)**

(54) **VERWENDUNG EINES SCHALENKATALYSATORS ZUR HERSTELLUNG VON ESSIGSÄURE DURCH GASPHASENOXIDATION VON UNGESÄTTIGTEN C 4-KOHLENWASSERSTOFFEN**

USE OF A SHELL CATALYST FOR PRODUCING ACETIC ACID BY GAS PHASE OXIDATION OF UNSATURATED C 4 HYDROCARBONS

UTILISATION D'UN CATALYSATEUR A COQUE POUR LA PREPARATION D'ACIDE ACETIQUE PAR OXYDATION EN PHASE GAZEUSE D'HYDROCARBURES C 4 INSATURES

(84) Benannte Vertragsstaaten:
**AT BE CH DE ES FI FR GB IT LI NL SE**

(30) Priorität: **28.11.1996 DE 19649426**

(43) Veröffentlichungstag der Anmeldung:
**27.10.1999 Patentblatt 1999/43**

(73) Patentinhaber: **Consortium für elektrochemische Industrie GmbH**
**81379 München (DE)**

(72) Erfinder:
  • **RUEDINGER, Christoph**
    **D-81479 München (DE)**
  • **EBERLE, Hans-Juergen**
    **D-81477 München (DE)**
  • **ZEITLER, Norbert**
    **D-80339 München (DE)**
  • **WOLLIN, Max**
    **D-81475 München (DE)**
  • **WITTMANN, Gudrun**
    **D-80636 München (DE)**

(74) Vertreter: **Schuderer, Michael, Dr. et al**
**Wacker-Chemie GmbH**
**Zentralabteilung Patente**
**Marken und Lizenzen**
**Hanns-Seidel-Platz 4**
**81737 München (DE)**

(56) Entgegenhaltungen:
EP-A- 0 021 325          EP-A- 0 609 750
DE-A- 1 442 590          DE-A- 4 442 346
US-A- 4 146 734

**Beschreibung**

Die Erfindung betrifft die Verwendung eines Schalenkatalysator zur Herstellung von Essigsäure durch Gasphasenoxidation von ungesättigten $C_4$-Kohlenwasserstoffen, sowie Verfahren zur Herstellung von Essigsäure durch Gasphasenoxidation von ungesättigten $C_4$-Kohlenwasserstoffen unter Verwendung des Schalenkatalysators.

Es ist bekannt, daß Essigsäure durch Gasphasenoxidation von $C_2$-, $C_3$- und $C_4$-Körpern mittels eines Katalysators hergestellt werden kann. Bisher konnte jedoch noch kein wirtschaftlich und betriebstechnisch voll befriedigendes Verfahren gefunden werden.

Die DE-B 1279011 beschreibt ein Verfahren zur Herstellung von Essigsäure durch katalytische Gasphasenoxidation von Buten mit Sauerstoff unter Verwendung von Aluminium- und Titanvanadatkatalysatoren. Diese Katalysatoren werden durch Ausfällen der Mischoxide aus den entsprechenden Lösungen hergestellt, wobei die Mischoxide gegebenenfalls noch mit inerten Materialien wie Kieselsäure vermischt werden können. Der Katalysator wird als feinteiliges Pulver in Wirbelbettreaktoren eingesetzt. Nachteilig bei derartigen Vollkontakt-Katalysatoren ist der hohe Grad an Totaloxidation.

Zur Verbesserung der Ausbeute derartiger Katalysatoren wird in der DE-A 2016681 vorgeschlagen, die Katalysatoren vor der Calcinierung mit einem Oxidationsmittel vorzubehandeln. In der DE-A 2354425 (US-A 3954857) wird zur Verbesserung der Selektivität die Behandlung des calcinierten Titan-Vanadium-Mischkatalysators mit Salzsäure vorgeschlagen. Die Katalysatoren werden als Vollkontakt, gegebenenfalls im Gemisch mit inerten Trägermaterialien wie Kieselsäure, eingesetzt.

Ein weiterer aus dem Stand der Technik bekannter Ansatzpunkt, zur Verbesserung der Aktivität von Titan-Vanadium-Mischkatalysatoren bei der Gasphasenoxidation von Butenen zu Essigsäure, ist der Einsatz von $TiO_2$ in definierter Kristallform oder mit definierter Oberfläche. In der DE-A 2026744 (US-A 3917682) werden Ti-V-Mischkatalysatoren beschrieben, deren $TiO_2$-Komponente überwiegend als Rutil vorliegt. Die Katalysatoren können in Pulverform oder zu Formkörpern gepreßt eingesetzt werden. Aus der US-A 4448897 sind Ti-V-Katalysatoren zur Butenoxidation bekannt, welche $TiO_2$ mit einer BET-Oberfläche größer 40 $m^2$/g enthalten. Die Katalysatoren werden ebenfalls in Pulverform oder als Preßling eingesetzt.

Aus dem Stand der Technik ist weiter bekannt, die Selektivität von Ti-V-Katalysatoren bei der Butenoxidation dadurch zu verbessern, daß man den Titandioxid-Anteil ganz oder teilweise durch andere Metalloxide substituiert. Die DE-A 2110876 (GB-A 1333306) beispielsweise beschreibt Katalysatoren, welche Oxide von Molybdän, Zinn und Vanadium als Aktivkomponenten enthalten. Die Katalysatoren werden in Pulverform eingesetzt, wobei der Mischoxid-Katalysator gegebenenfalls auch auf feinteilige Trägermaterialien wie Siliciumdioxid aufgetragen werden kann. Aus der US-A 4146734 ist bekannt, Vanadium-Mischoxide einzusetzen, welche mit Cer und weiteren Übergangsmetalloxiden dotiert sind. Der Katalysator wird als feinteilges Granulat eingesetzt, kann aber auch als Präzipitat auf feinteilige, inerte Träger aufgetragen werden.

Aus der DE-A 2235103 sind Ti-V-Mischoxid-Katalysatoren zur Gasphasenoxidation von Butenen in Form von Trägerkatalysatoren bekannt, bei denen ein vorgeformter poröser Träger mit der Mischlösung der Katalysatorkomponenten getränkt wird.

Allen diesen Verfahren ist gemeinsam, daß es sich um Vollkontakt-Katalysatoren handelt, bei denen die Aktivkomponenten selbst als Pulver oder Preßlinge eingesetzt werden, oder mit feinteiligen Trägermaterialien verdünnt als Pulver oder Preßling eingesetzt werden. Als Vollkontakte sind auch mit Aktivkomponente durchtränkte poröse Träger gemäß DE-A 2235103 zu verstehen, da auch hier das gesamte Katalysatorvolumen katalytisch aktiv ist. Nachteilig bei Vollkontakt-Katalysatoren ist der hohe Grad an Totaloxidation und die bei hohen Umsätzen schlechte Beherrschbarkeit der Butenoxidation.

Es bestand daher die Aufgabe, ein Verfahren zur Herstellung von Essigsäure durch Gasphasenoxidation von ungesättigten $C_4$-Kohlenwasserstoffen zur Verfügung zu stellen, welche zu besserer Ausbeute und besserem Betriebsverhalten bei der Oxidationsreaktion führen.

Es wurde gefunden, daß sich für die Herstellung von Essigsäure durch Gasphasenoxidation von ungesättigten Kohlenwasserstoffen mit vier Kohlenstoffatomen besonders Schalenkatalysatoren eignen, bei denen die aktive Masse als dünne Schicht auf einem unporösen Trägerkörper aufgebracht ist.

Gegenstand der Erfindung ist die Verwendung eines Schalenkatalysator zur Herstellung von Essigsäure durch Gasphasenoxidation von ungesättigten $C_4$-Kohlenwasserstoffen bestehend aus einem inerten unporösen Trägerkörper und einer auf die äußere Oberfläche des Trägerkörpers aufgebrachten katalytisch aktiven Mischoxidmasse, welche

a) ein oder mehrere Oxide aus der Gruppe Titandioxid, Zirkoniumdioxid, Zinndioxid, Aluminiumoxid und

b) 0.1 bis 1.5 Gew%, bezogen auf das Gewicht der Komponente a) und pro $m^2$/g spezifischer Oberfläche der Komponente a), Vanadiumpentoxid enthält.

Die Angabe Gew% bezogen auf das Gewicht der Komponente a) und pro $m^2$/g spezifischer Oberfläche der Komponente a) bedeutet dabei, daß der einzusetzende Gewichtsanteil der Komponente b) von der spezifischen Oberfläche der Komponente a) abhängig ist. So ist beispielsweise bei einer spezifischen Oberfläche der Komponente a) von 10 $m^2$/g der Anteil der Komponente b) 1 bis 15 Gew%, bezogen auf das Gewicht der Komponente a).

Geeignet ist $TiO_2$ sowohl in Rutil- als auch in Anatas-Form und deren Gemische. Bevorzugt wird als Komponente a) Titandioxid mit einer BET-Oberfläche von 20 bis 400 $m^2$/g, vorzugsweise 70 bis 300 $m^2$/g. Werden Gemische von Titandioxid mit Zirkoniumdioxid oder Zinndioxid eingesetzt, kann der Titandioxidanteil zu 5 bis 95 Gew%, vorzugsweise 5 bis 50 Gew%, durch Zirkoniumdioxid, Aluminiumoxid oder Zinndioxid ersetzt werden.

Als zusätzliche Komponente a) können noch ein oder mehrere Oxide der Metalle aus der Gruppe Hafnium, Niob, Wolfram, Lanthan und Cer enthalten sein. Bei der Dotierung der Komponente a) mit den genannten Oxiden sind diese im allgemeinen in einer Menge von 1 bis 15 Gew%, bezogen auf das Gesamtgewicht der Komponente a), enthalten.

Der Anteil der Komponente b) beträgt vorzugsweise 0.1 bis 0.5 Gew%, besonders bevorzugt 0.1 bis 0.2 Gew%, jeweils bezogen auf das Gewicht der Komponente a) und pro $m^2$/g spezifischer Oberfläche der Komponente a)

Bei der Komponente b) kann gegebenenfalls ein Teil des Vanadiumpentoxids, vorzugsweise 10 bis 90 Gew%, durch ein oder mehrere Oxide von Molybdän, Chrom und Antimon ersetzt werden. Gegebenenfalls können als zusätzliche Komponente b) noch ein oder mehrere Oxide von Alkalimetallen, Elementen der 5. und 6. Hauptgruppe des Periodensystems der Elemente (PSE) und der Übergangsmetalle enthalten sein. Beispiele hierfür sind die Oxide von Lithium, Natrium, Kalium, Rubidium, Caesium, Phosphor, Wismut, Schwefel, Selen, Tellur, Mangan, Eisen, Cobalt, Palladium, Kupfer, Silber, Gold, Zink und Cadmium. Im allgemeinen beträgt die Menge dieser Dotierstoffe 0.05 bis 15 Gew%, berechnet als Oxide und bezogen auf das Gesamtgewicht der Komponente b). Der Anteil an Alkalimetalloxiden und Edelmetalloxiden beträgt dabei vorzugsweise 0.05 bis 1.0 Gew%.

Bevorzugt werden Zusammensetzungen mit hoher Oberfläche der Komponente a) von 70 bis 300 $m^2$/g, wobei gegebenenfalls noch Zinn- oder Wolframoxid enthalten sein können, und mit einer Komponente b), welche mit Mo, Cr, Sb und/oder Au dotiert ist.

Die katalytisch aktive Mischoxidmasse kann gegebenenfalls noch 10 bis 50 Gew%, bezogen auf das Gesamtgewicht der katalytisch aktiven Mischoxidmasse, inerte Verdünnungsmittel wie Siliciumdioxid, Siliciumcarbid enthalten.

Die katalytisch aktive Mischoxidmasse ist in einem Anteil von 1 bis 40 Gew%, vorzugsweise 5 bis 25 Gew%, jeweils bezogen auf das Gesamtgewicht aus Trägerkörper und aktiver Masse, als Schale auf die äußere Oberfläche des Trägerkörpers aufgebracht. Die Schichtdicke beträgt im allgemeinen 10 bis 1000 µm, vorzugsweise 100 bis 500 µm. Der Schalenkatalysator kann auch mehrere, sich in der Zusammensetzung unterscheidende, Schichten enthalten. Es können auch ein oder mehrere Bestandteile der Aktivkomponente a) und b) in unterschiedlicher Konzentration in den einzelnen Schichten enthalten sein.

Im allgemeinen wird die katalytisch aktive Mischoxidmasse in einer Schicht aufgetragen. Zur Beeinflußung der Katalysatoraktivität und zur Verbesserung der Haftung am Trägerkörper können auch zwei oder mehr Schichten aufgetragen werden.

Bevorzugte Ausführungsformen mit mehreren Schichten sind solche in denen die innere Schicht nur Komponente a) enthält und die äußere Schicht die Komponenten a) und b). Bevorzugte Mehrschicht-Ausführungsformen sind auch solche, in denen die innere und äußere Schicht jeweils die Komponenten a) und b) enthalten, wobei vorzugsweise für die innere Schicht eine höhere spezifische Oberfläche für die Komponente a) gewählt wird, als für die äußere Schicht.

Geeignete Materialien für den inerten, unporösen Trägerkörper sind alle unter den Betriebsbedingungen der Gasphasenoxidation sich inert verhaltenden und über den Betriebszeitraum stabilen unporösen Materialien. Beispiele hierfür sind Steatit, Duranit, Siliciumcarbid, Magnesiumoxid, Siliciumoxid, Silikate, Aluminate, Metalle wie Edelstahl, sowie gegebenenfalls Mischungen aus diesen Stoffen. Bevorzugt wird keramisches Material wie Steatit.

Der Trägerkörper ist unporös, wobei unter unporös zu verstehen ist, daß die BET-Oberfläche des Trägerkörpers < 1 $m^2$/g und die Porosität < 0.1 beträgt, mit

$$\text{Porosität} = [1 - (\text{Dichte}_{\text{Formkörper}} / \text{Dichte}_{\text{Substanz}})] \, .$$

Die Formgestalt des inerten, unporösen Trägerkörpers ist beliebig. Beispiele für geeignete Formen sind Kugeln, Zylinder, Quader, Tori, Sättel, Spindeln, Wendeln. Die Grundkörper können auch eine oder mehrere Ausnehmungen wie Mulden, Rillen, Löcher, oder auch abstehende Teile wie Zapfen, Spitzen, Stege besitzen. Weitere Beispiele sind Ringe, Ringsegmente, Steg-Ringe, durchbohrte Kugeln, Kugelsegmente. Ebenfalls als Träger geeignet sind geordnete Packungen wie Monolithe oder Kreuzkanalstrukturen. Bevorzugt sind Trägerformen mit möglichst hoher geometrischer Oberfläche pro Volumen, beispielsweise Ringe.

Die Abmessungen der Trägerkörper sind durch die Reaktoren zur Gasphasenoxidation vorgegeben. Im allgemeinen haben die Formkörper eine Länge bzw. einen Durchmesser von 2 bis 20 mm. Die Wanddicke, beispielsweise im Fall von Ringen oder Hohlzylindern, beträgt zweckmäßigerweise 0.1 bis 4 mm.

Zur Herstellung der Schalenkatalysatoren wird die katalytisch aktive Mischoxidmasse auf bekannte Art und Weise auf den Trägerkörper aufgebracht, beispielsweise durch Beschichten der Träger im Drehrohrofen mit einer wässerigen Aufschlämmung oder Dragieren. Vorteilhaft ist es, die Vormischung der aktiven Masse mit einem Binder zu versehen, der nach dem Aufbringen in der aktiven Schicht verbleibt um deren mechanische Stabilität zu verbessern. Besonders vorteilhaft ist es, die wässerige Suspension der Aktivkomponenten mit einer wässerigen Copolymerdispersion, vorzugsweise aus Vinylacetat/Vinyllaurat in einer Menge von 5 bis 40 Gew%, bezogen auf den Feststoffgehalt der Dispersion und die Summe der Trockengewichte von aktiver Masse und Dispersion, zu vermischen und diese Mischung in einem Sprühtrocknungsschritt auf die inerten, nicht porösen Trägerkörper aufzubringen.

Durch Wiederholen dieses Schrittes mit weiteren Sprühsuspensionen anderer Zusammensetzung können Katalysatoren mit schichtartigem Aufbau der aktiven Katalysatorschale hergestellt werden. Werden eine bzw. mehrere Komponenten der aktiven Masse während des Aufbringvorgangs in sich zeitlich ändernden Mengen zugeführt, werden katalytisch aktive Schichten erhalten, die eine stetige Änderung der Zusammensetzung entlang der Dickenachse zeigen.

Ein weiterer Gegenstand der Erfindung ist ein Verfahren zur Herstellung von Essigsäure durch Gasphasenoxidation von ungesättigten $C_4$-Kohlenwasserstoffen unter Verwendung des Schalenkatalysators. Dazu wird ein Gasgemisch, enthaltend Sauerstoff oder ein sauerstoffhaltiges Gas, bevorzugt Luft, ein oder mehrere $C_4$-Kohlenwasserstoffe, bevorzugt Buten, Wasserdampf und gegebenenfalls ein Inertgas, bei erhöhter Temperatur an dem Schalenkatalysator umgesetzt.

Die Gasphasenoxidation wird in gekühlten Rohrreaktoren, welche mit dem Schalenkatalysator befüllt sind und vom Reaktionsgemisch durchströmt werden, durchgeführt. Übliche Festbettreaktoren sind senkrecht angeordnete Rohrbündelreaktoren mit Rohrlängen von 1 m bis 10 m, einem Rohr-Innendurchmesser von 10 bis 35 mm und einer Wandstärke von 1 bis 4 mm. Als Wärmetauschermedien zur Kühlung sind besonders Wasser, Wärmeträgeröle und eutektische Salzschmelzen (z.B. $KNO_3/NaNO_2$) geeignet.

Die Reaktionsrohre können gegebenenfalls mit Schalenkatalysatoren unterschiedlicher Gestalt und Dimension sowie unterschiedlicher Zusammensetzung der Aktivkomponenten bzw. Schalen befüllt werden. Dabei können die Schalenkatalysatoren als statistische Mischung oder zonenweise in die Reaktionsrohre eingebracht werden.

Geeignete Edukte sind ungesättigte Kohlenwasserstoffe mit vier Kohlenstoffatomen oder Gasgemische die Kohlenwasserstoffe mit vier Kohlenstoffatomen enthalten. Butene liefern höhere Ausbeuten als Butadiene. Bevorzugt sind Butenisomere, besonders bevorzugt 1-Buten, 2-Butene und deren Gemische.

Ein Vorteil der erfindungsgemäßen Verfahrensweise der Gasphasenoxidation von ungesättigten $C_4$-Kohlenwasserstoffen unter Verwendung des Schalenkatalysators besteht darin, daß auch Gasgemische eingesetzt werden können, welche Verbindungen enthalten, die nicht oder nur in geringem Maße oder in schlechten Ausbeuten zu Essigsäure reagieren. Es können somit auch billige Rohstoffgemische aus Raffinerien wie "$C_4$-Schnitt" (überwiegender Anteil an Butadien und i-Buten), "Raffinat 1" (überwiegender Anteil an i-Buten), "Raffinat 2" (überwiegender Anteil an 1-Buten und 2-Butenen) als Ausgangsmaterial eingesetzt werden. Die genannten Rohstoffgemische können gegebenenfalls vor deren Einsatz auch einem Hydrier- oder Reinigungsschritt unterzogen werden.

Die Reaktionstemperatur für die Oxidation der Buten/Sauerstoff (Luft) /Wasserdampf-Reaktionsgemische beträgt im allgemeinen 100°C bis 400°C, vorzugsweise 150°C bis 300°C. Die Reaktion kann bei dem Staudruck, der sich durch die Durchströmung des Katalysatorbettes ergibt oder unter erhöhtem Druck durchgeführt werden. Bevorzugt wird bei 0.2 bis 30 bar Überdruck gearbeitet.

Das Volumenmischungsverhältnis Buten(mischung)/Sauerstoff(Luft) beträgt im allgemeinen 0.2/99.5 bis 15/85, bevorzugt 1/99 bis 5/95. Das Volumenmischungsverhältnis Buten(mischung)/Wasserdampf beträgt im allgemeinen 1/1 bis 1/50, bevorzugt 1/5 bis 1/25. Die Raumgeschwindigkeit des Gasgemisches im Reaktor liegt bei 400 bis 10000 $h^{-1}$, vorzugsweise 600 bis 6000 $h^{-1}$ (Normbedingungen).

Nach der Umsetzung wird die gebildete Essigsäure durch Abkühlen und Niederschlagen oder durch Absorption in einem geeigneten Lösungsmittel abgetrennt. Die abgetrennte Essigsäure wird mit geeigneten Verfahren, beispielsweise Destillation oder Extraktion weiter gereinigt. Das Abgas kann im Kreislauf zurückgeführt werden.

Die nachfolgenden Beispiele sollen die Erfindung, ohne diese einzuschränken, näher erläutern.

Katalysatorenherstellung:

Die Katalysatoren wurden, in den in den Beispielen angegebenen Mengen, durch Vermahlen der Aktivkomponenten unter Zusatz von Wasser, anschließender Zugabe einer Copolymerdispersion aus Vinylacetat und Vinyllaurat mit 50 Gew% Feststoffanteil, und Versprühen der fertigen Suspension, unter Verdampfung des Wassers, auf 1000 g Steatit-Kugeln (4mm Durchmesser, BET-Oberfläche < 0.01, Porosität < 0.01) aufgebracht.

Testung der Katalysatoren:

Die Katalysatoren wurden in ein Reaktionsrohr mit einem Innendurchmesser von 12.5 mm gefüllt. Das Rohr wurde von außen mit einer zwangsumgewälzten Salzschmelze gekühlt. Soweit nichts anderes erwähnt wurden alle Umsetzungen bei $1 \times 10^5$ Pa Überdruck durchgeführt.

Es wurden die in den Beispielen genannten Katalysatormengen in den Reaktor (Füllhöhe von 1400 mm) gefüllt. Falls nicht anderes in den Beispielen angegeben, wurde der Katalysator vor dem Betrieb für 6 h bei 410°C und einem Luftfluß von 220 Nl/h im Reaktionsrohr getempert. Das Reaktionsgas bestand, soweit nicht anders angegeben, aus 80 Nl/h Luft, 0.81 Nl/h 1-Buten und 16.2 Nl/h Wasserdampf.

Die Ausbeute wird nach folgender Gleichung bestimmt:

$$\text{Essigsäureausbeute [Gew\%]} = (\text{kg abgeschiedene Essigsäure / kg eingesetztes Edukt}) \times 100$$

Die Katalysatorzusammensetzungen sowie die Testbedingungen und Testergebnisse sind in Tabelle 1 zusammengefaßt.

Beispiel 1: (Zweischalenkatalysator) :

1. Sprühsuspension: 70.42 g Titandioxid (> 70 % Anatas-Modifikation) mit einer BET-Oberfläche von 48 $m^2$/g wurden mit 7.43 g Titandioxid (100 % Anatasmodifikation, BET 8 $m^2$/g) und mit 14.67 g $V_2O_5$ und 900 ml vollentsalztem Wasser 20 h in einer Kugelmühle vermahlen. Zu der homogenen Suspension wurden danach 29 g einer Copolymerdispersion aus Vinylacetat und Vinyllaurat mit 50 Gew% Feststoffanteil zugegeben und innig verrührt. 2. Sprühsuspension: 5 g Titandioxid (100 % Anatasmodifikation, BET 8 $m^2$/g) und 3 g einer Copolymerdispersion aus Vinylacetat/Vinyllaurat mit 50 Gew% Feststoffanteil und 100 ml vollentsalztem Wasser wurden innig verrührt. Auf 1000 g 4 mm Steatit-Kugeln wurde zuerst die 2. Sprühsuspension aufgebracht und getrocknet. Danach wurde die 1. Sprühsuspension aufgebracht und getrocknet.

202 g Katalysator wurden in den Reaktor (Füllhöhe von 1400 mm) gefüllt. Bei einer Salzbadtemperatur von 213.5 °C wurde ein Umsatz von 88.6 % erreicht, die Essigsäureausbeute betrug 116.25 Gew%.

Beispiel 2: (Einschalenkatalysator,niedere Oberfläche):

211 g Titandioxid (> 70 % Anatas-Modifikation) mit einer BET-Oberfläche von 48 $m^2$/g wurden mit 44 g $V_2O_5$ und 1500 ml vollentsalztem Wasser 20 h in einer Kugelmühle vermahlen und nach Zugabe des Bindemittels auf die Steatit-Kugeln aufgebracht. 200 g Katalysator wurden in den Reaktor (Füllhöhe von 1400 mm) gefüllt. Bei einer Salzbadtemperatur von 198 °C wurde ein Umsatz von 88 % erreicht, die Essigsäureausbeute betrug 116.5 Gew.-%.

Beispiel 3 (hoher Wasserdampfanteil):

Es wurde analog Beispiel 2 vorgegangen, mit dem Unterschied, daß das Reaktionsgas aus 100 Nl/h Luft, 1.01 Nl/h 1-Buten und 50.5 Nl/h Wasserdampf bestand. Bei einer Salzbadtemperatur von 197°C wurde ein Umsatz von 75 erreicht, die Essigsäureausbeute betrug 103 Gew%.

Beispiel 4 (hohe Schichtdicke):

297.1 g Titandioxid (> 70 % Anatas-Modifikation) mit einer BET-Oberfläche von 48 $m^2$/g wurden mit 51.3 g $V_2O_5$ und 1500 ml vollentsalztem Wasser 19 h in einer Kugelmühle vermahlen und nach Zugabe des Bindemittels auf die Steatit-Kugeln aufgebracht. 177 g Katalysator wurden in den Reaktor (Füllhöhe von 1400 mm) gefüllt. Das Reaktionsgas enthielt 24.8 Nl/h Wasserdampf. Bei einer Salzbadtemperatur von 196°C wurde ein Umsatz von 95 % erreicht, die Essigsäureausbeute betrug 113.5 Gew%.

Beispiel 5 (hohe Vanadiummenge) :

135 g Titandioxid (Anatas-Modifikation) mit einer BET-Oberfläche von 75 $m^2$/g wurden mit 120 g $V_2O_5$ und 1400 ml vollentsalztem Wasser 24 h in einer Kugelmühle vermahlen und nach Zugabe des Bindemittels auf die Steatit-Kugeln aufgebracht.

168 g Katalysator wurden in den Reaktor (Füllhöhe von 1400 mm) gefüllt. Das Reaktionsgas enthielt 24.8 Nl/h Wasserdampf. Bei einer Salzbadtemperatur von 189°C wurde ein Umsatz von 92 % erreicht, die Essigsäureausbeute betrug 105 Gew%.

Beispiel 6 (Wolframdotierung) :

186.2 g Titandioxid (Anatas-Modifikation mit 10 Gew% $WO_3$) mit einer BET-Oberfläche von 75 m$^2$/g wurden mit 68.9 g $V_2O_5$ und 700 ml vollentsalztem Wasser 120 h in einer Kugelmühle vermahlen und nach Zugabe des Bindemittels auf die Steatit-Kugeln aufgebracht.

192 g Katalysator wurden in den Reaktor (Füllhöhe von 1400 mm) gefüllt. Bei einer Salzbadtemperatur von 179°C wurde ein Umsatz von 95 % erreicht, die Hot-Spot-Temperatur betrug 190°C, die Essigsäureausbeute betrug 116.2 Gew%.

Beispiel 7 (geringe $C_4$-Konzentration) :

Es wurde analog Beispiel 5 vorgegangen, mit dem Unterschied, daß das Reaktionsgas aus 206 Nl/h Luft, 0.62 Nl/h 1-Buten und 42 Nl/h Wasserdampf bestand. Bei einer Salzbadtemperatur von 192.5°C wurde ein Umsatz von 96 % erreicht, die Essigsäureausbeute betrug 128.1 Gew.-%.

Beispiel 8 (Einschalenkatalysator mit sehr hoher Oberfläche):

186.37 g Titandioxid (Anatas-Modifikation) mit einer BET-Oberfläche von 250 m$^2$/g wurden mit 68 g $V_2O_5$ und 1500 ml vollentsalztem Wasser 18 h in einer Kugelmühle vermahlen und nach Zugabe des Bindemittels auf die Steatit-Kugeln aufgebracht.

153.4 g Katalysator wurden in den Reaktor (Füllhöhe von 1400 mm) gefüllt.

Bei einer Salzbadtemperatur von 174°C wurde ein Umsatz von 96.2 % erreicht, die Essigsäureausbeute betrug 133 Gew%.

Beispiel 9 (Caesium/Phosphor-Dotierung):

217.5 g Titandioxid (>70% Anatas-Modifikation) mit einer BET-Oberfläche von 48 m$^2$/g wurden mit 37.6 g $V_2O_5$, 1.6 g Caesiumcarbonat, 4.8 g Ammoniumdihydrogenphosphat und 1000 ml vollentsalztem Wasser 48 h in einer Kugelmühle vermahlen und nach Zugabe des Bindemittels auf die Steatit-Kugeln aufgebracht. 166.8 g Katalysator wurden in den Reaktor (Füllhöhe von 1400 mm) gefüllt.

Bei einer Salzbadtemperatur von 221.5°C wurde ein Umsatz von 94 % erreicht, die Hot-Spot-Temperatur betrug 223°C, die Essigsäureausbeute betrug 105.5 Gew%.

Beispiel 10 (Molybdändotierung):

219.98 g Titandioxid (Anatas-Modifikation) mit einer BET-Oberfläche von 44.4 m$^2$/g wurden mit 31.33 g $V_2O_5$, 6.25 g Molybdäntrioxid und 1000 ml vollentsalztem Wasser 22 h in einer Kugelmühle vermahlen und nach Zugabe des Bindemittels auf die Steatit-Kugeln aufgebracht.

167 g Katalysator wurden in den Reaktor (Füllhöhe von 1400 mm) gefüllt.

Bei einer Salzbadtemperatur von 194°C wurde ein Umsatz von 93 % erreicht, die Essigsäureausbeute betrug 112.4 Gew%.

Beispiel 11 (Zweischalenkatalysator) :

1. Sprühsuspension: 101.65 g Titandioxid (100 % Anatas-Modifikation) mit einer BET-Oberfläche von 75 m$^2$/g wurden mit 27.1 g $V_2O_5$ und 500 ml vollentsalztem Wasser 20 h in einer Kugelmühle vermahlen. Zu der homogenen Suspension wurden danach 43.50 g einer Copolymerdispersion aus Vinylacetat und Vinyllaurat mit 50 Gew% Feststoffanteil zugegeben und innig verrührt.

2. Sprühsuspension: 122.6 g Titandioxid (100 % Anatasmodifikation, BET 17 m$^2$/g), 7.50 g Vanadiumpentoxid und 500 ml vollentsalztes Wasser wurden 20 h in einer Kugelmühle vermahlen. Zu der homogenen Suspension wurden danach 43.49 g einer Copolymerdispersion aus Vinylacetat und Vinyllaurat mit 50 Gew% Feststoffanteil zugegeben und innig verrührt.

Auf 1000 g 4mm Steatit-Kugeln wurde zuerst die 1. Sprühsuspension aufgebracht. Danach wurde die 2. Sprühsuspension aufgebracht.
167 g Katalysator wurden in den Reaktor (Füllhöhe von 1400 mm) gefüllt.
Bei einer Salzbadtemperatur von 185°C wurde ein Umsatz von 92 % erreicht, die Essigsäureausbeute betrug 116 Gew%.

Beispiel 12 (Mo/Na-Dotierung):

100.0 g Titandioxid (100% Anatas-Modifikation) mit einer BET-Oberfläche von 15 $m^2$/g wurden mit 5.32 g $V_2O_5$, 1.065 g Molybdäntrioxid, 0.245 g Natriumcarbonat und 1000 ml vollentsalztem Wasser 20 h in einer Kugelmühle vermahlen und nach Zugabe des Bindemittels auf die Steatit-Kugeln aufgebracht. 202 g Katalysator wurden in den Reaktor (Füllhöhe von 1400 mm) gefüllt.
Bei einer Salzbadtemperatur von 252°C wurde ein Umsatz von 92 % erreicht, die Essigsäureausbeute betrug 96.4 Gew%.

Beispiel 13 (geringer Vanadiumanteil) :

225 g Titandioxid (100% Anatas-Modifikation) mit einer BET-Oberfläche von 75 $m^2$/g wurden mit 30 g $V_2O_5$ und 1500 ml vollentsalztem Wasser 35 h in einer Kugelmühle vermahlen und nach Zugabe des Bindemittels auf 1000 g 4mm Steatit-Kugeln aufgebracht.
158 g Katalysator wurden in den Reaktor (Füllhöhe von 1400 mm) gefüllt. Bei einer Salzbadtemperatur von 179°C wurde ein Umsatz von 95 % erreicht, die Essigsäureausbeute betrug 121.3 %.

Beispiel 14 (Schwefeldotierung) :

188 g Titandioxid (Anatas-Modifikation) mit einer BET-Oberfläche von 140 $m^2$/g und einem Sulfatgehalt von 4.6 Gew.-% wurden mit 69 g $V_2O_5$ und 1500 ml vollentsalztem Wasser 100 h in einer Kugelmühle vermahlen und nach Zugabe des Bindemittels auf die Steatit-Kugeln aufgebracht.
161 g Katalysator wurden in den Reaktor (Füllhöhe von 1400 mm) gefüllt.
Bei einer Salzbadtemperatur von 172.5°C wurde ein Umsatz von 95 % erreicht, die Essigsäureausbeute betrug 130 Gew%.

Beispiel 15 (hoher Mo-Gehalt) :

225 g Titandioxid (Anatas-Modifikation) mit einer BET-Oberfläche von 75 $m^2$/g wurden mit 10 g $V_2O_5$, 20 g $MoO_3$ und 1500 ml vollentsalztem Wasser 68 h in einer Kugelmühle vermahlen und nach Zugabe des Bindemittels auf die Steatit-Kugeln aufgebracht.
162 g Katalysator wurden in den Reaktor (Füllhöhe von 1400 mm) gefüllt.
Bei einer Salzbadtemperatur von 183.5°C wurde ein Umsatz von 94.5 % erreicht, die Essigsäureausbeute betrug 120 Gew%.

Beispiel 16 (Antimondotierung) :

225 g Titandioxid (Anatas-Modifikation) mit einer BET-Oberfläche von 75 $m^2$/g wurden mit 26.3 g $V_2O_5$, 10.5 g $Sb_2O_3$ und 2000 ml vollentsalztem Wasser 24 h in einer Kugelmühle vermahlen und nach Zugabe des Bindemittels auf die Steatit-Kugeln aufgebracht.
166 g Katalysator wurden in den Reaktor (Füllhöhe von 1400 mm) gefüllt.
Bei einer Salzbadtemperatur von 175°C wurde ein Umsatz von 94.4 % erreicht, die Essigsäureausbeute betrug 142 Gew%.

Beispiel 17 (Wismutdotierung) :

225 g Titandioxid (100 % Anatas-Modifikation) mit einer BET-Oberfläche von 75 $m^2$/g wurden mit 26.3 g $V_2O_5$, 10.5 g $Bi_2O_3$ und 1500 ml vollentsalztem Wasser 48 h in einer Kugelmühle vermahlen und nach Zugabe des Bindemittels auf die Steatit-Kugeln aufgebracht.
151 g Katalysator wurden in den Reaktor (Füllhöhe von 1400 mm) gefüllt.

Bei einer Salzbadtemperatur von 179°C wurde ein Umsatz von 90.2 % erreicht, die Essigsäureausbeute betrug 120 Gew%.

Beispiel 18 (Tellurdotierung) :

225 g Titandioxid (100% Anatas-Modifikation) mit einer BET-Oberfläche von 75 $m^2$/g wurden mit 26.3 g $V_2O_5$, 10.5 g $TeO_2$ und 2000 ml vollentsalztem Wasser 47 h in einer Kugelmühle vermahlen und nach Zugabe des Bindemittels auf die Steatit-Kugeln aufgebracht.
159 g Katalysator wurden in den Reaktor (Füllhöhe von 1400 mm) gefüllt.
Bei einer Salzbadtemperatur von 203°C wurde ein Umsatz von 88 % erreicht, die Essigsäureausbeute betrug 103 Gew%.

Beispiel 19 (Mangandotierung) :

225 g Titandioxid (100 % Anatas-Modifikation) mit einer BET-Oberfläche von 75 $m^2$/g wurden mit 26.3 g $V_2O_5$, 10.5 g $MnO_2$ und 1500 ml vollentsalztem Wasser 19 h in einer Kugelmühle vermahlen und nach Zugabe des Bindemittels auf die Steatit-Kugeln aufgebracht.
158 g Katalysator wurden in den Reaktor (Füllhöhe von 1400 mm) gefüllt.
Bei einer Salzbadtemperatur von 194°C wurde ein Umsatz von 90 % erreicht, die Essigsäureausbeute betrug 111.3 Gew%.

Beispiel 20 (Kupfer-Dotierung):

225 g Titandioxid (100 % Anatas-Modifikation) mit einer BET-Oberfläche von 75 $m^2$/g wurden mit 26.3 g $V_2O_5$, 1.42 g $Cu(NO_3)_2 \cdot X3H_2O$ und 1200 ml vollentsalztem Wasser 19 h in einer Kugelmühle vermahlen und nach Zugabe des Bindemittels auf die Steatit-Kugeln aufgebracht.
159 g Katalysator wurden in den Reaktor (Füllhöhe von 1400 mm) gefüllt.
Bei einer Salzbadtemperatur von 180°C wurde ein Umsatz von 94 % erreicht, die Essigsäureausbeute betrug 120 Gew%.

Beispiel 21 (Zinkdotierung) :

225 g Titandioxid (100 % Anatas-Modifikation) mit einer BET-Oberfläche von 75 $m^2$/g wurden mit 26.3 g $V_2O_5$, 1.11 g Zink(II)acetat-dihydrat und 1500 ml vollentsalztem Wasser 43 h in einer Kugelmühle vermahlen und nach Zugabe des Bindemittels auf die Steatit-Kugeln aufgebracht.
157 g Katalysator wurden in den Reaktor (Füllhöhe von 1400 mm) gefüllt.
Bei einer Salzbadtemperatur von 178.5°C wurde ein Umsatz von 94.6 % erreicht, die Essigsäureausbeute betrug 124.2 Gew%.

Beispiel 22 (Golddotierung) :

225 g Titandioxid (100 % Anatas-Modifikation) mit einer BET-Oberfläche von 75 $m^2$/g wurden mit 26.3 g $V_2O_5$, 0.94 g Tetrachlorogoldsäure und 1500 ml vollentsalztem Wasser 46 h in einer Kugelmühle vermahlen und nach Zugabe des Bindemittels auf die Steatit-Kugeln aufgebracht.
162 g Katalysator wurden in den Reaktor (Füllhöhe von 1400 mm) gefüllt.
Bei einer Salzbadtemperatur von 182°C wurde ein Umsatz von 95.8 % erreicht, die Essigsäureausbeute betrug 128.4 Gew%.

Beispiel 23 (Chromdotierung) :

225 g Titandioxid (100 % Anatas-Modifikation) mit einer BET-Oberfläche von 75 $m^2$/g wurden mit 24 g $V_2O_5$, 2.9 g Chromtrioxid und 1500 ml vollentsalztem Wasser 22 h in einer Kugelmühle vermahlen und nach Zugabe des Bindemittels auf die Steatit-Kugeln aufgebracht.
174.8 g Katalysator wurden in den Reaktor (Füllhöhe von 1400 mm) gefüllt.
Bei einer Salzbadtemperatur von 184°C wurde ein Umsatz von 95 % erreicht, die Essigsäureausbeute betrug 128 Gew%.

Beispiel 24 (Ti/Sn-Katalysator) :

200 g Titandioxid (100 % Anatas-Modifikation) mit einer BET-Oberfläche von 75 m$^2$/g wurden mit 70 g Zinntetrachlorid-Pentahydrat und 26.3 g $V_2O_5$ und 1500 ml vollentsalztem Wasser 46 h in einer Kugelmühle vermahlen und nach Zugabe des Bindemittels auf die Steatit-Kugeln aufgebracht.
160.1 g Katalysator wurden in den Reaktor (Füllhöhe von 1400 mm) gefüllt.
Bei einer Salzbadtemperatur von 191°C wurde ein Umsatz von 94 % erreicht, die Essigsäureausbeute betrug 130 Gew%.

Beispiel 25 (Ti/Zr-Katalysator) :

171 g eines über ein Sol-Gel-Verfahren hergestellten Ti/Zr-Mischoxides (9 Gew% $ZrO_2$) mit einer BET-Oberfläche von 75 m$^2$/g wurden mit 15.2 g $V_2O_5$, 3.8 g $MoO_3$, 9.1 g $Sb_2O_3$ und 1000 ml vollentsalztem Wasser 14 h in einer Kugelmühle vermahlen und nach Zugabe des Bindemittels auf 760 g der Steatit-Kugeln aufgebracht.
162.4 g Katalysator wurden in den Reaktor (Füllhöhe von 1400 mm) gefüllt.
Bei einer Salzbadtemperatur von 201°C wurde ein Umsatz von 93 % erreicht, die Essigsäureausbeute betrug 112 Gew%.

Beispiel 26 (Ti/Zr-Katalysator) :

171 g eines über ein Sol-Gel-Verfahren hergestellten Ti/Zr-Mischoxides (9 Gew% $ZrO_2$) mit einer BET-Oberfläche von 110 m$^2$/g wurden mit 36.8 g $V_2O_5$, 9.2 g $MoO_3$, 21.3 g $Sb_2O_3$ und 1000 ml vollentsalztem Wasser 14 h in einer Kugelmühle vermahlen und nach Zugabe des Bindemittels auf 760 g der Steatit-Kugeln aufgebracht.
155.5 g Katalysator wurden in den Reaktor (Füllhöhe von 1400 mm) gefüllt.
Bei einer Salzbadtemperatur von 199°C wurde ein Umsatz von 95 % erreicht, die Essigsäureausbeute betrug 115 Gew%.

Beispiel 27 (Niob-Dotierung) :

151 g eines über ein Sol-Gel-Verfahren hergestellten Ti/Nb-Mischoxides (9 Gew% $Nb_2O_5$) mit einer BET-Oberfläche von 70 m$^2$/g wurden mit 14.5 g $V_2O_5$, 3.6 g $MoO_3$, 8.7 g $Sb_2O_3$ und 800 ml vollentsalztem Wasser 14 h in einer Kugelmühle vermahlen und nach Zugabe des Bindemittels auf 700 g der Steatit-Kugeln aufgebracht.
169.5 g Katalysator wurden in den Reaktor (Füllhöhe von 1400 mm) gefüllt.
Bei einer Salzbadtemperatur von 219°C wurde ein Umsatz von 96 % erreicht, die Essigsäureausbeute betrug 105 Gew%.

Vergleichsbeispiel 1 (Vollkontakt-Katalysator) :

200 g Titandioxid (100 % Anatas-Modifikation) mit einer BET-Oberfläche von 8 m$^2$/g wurden mit 8.1 g $V_2O_5$ und 10 g Graphit vermischt, vermahlen, gesiebt und zu zylindrischen Tabletten (4x4 mm) verpreßt.
155 g Katalysator wurden in den Reaktor (Füllhöhe von 1400 mm) gefüllt.
Bei einer Salzbadtemperatur von 247°C wurde ein Umsatz von 90 % erreicht, die Essigsäureausbeute betrug 77 Gew%.

Vergleichsbeispiel 2 (DE-A 2235103) :

Ein poröser (Porosität = 0.65) alpha-Aluminiumoxidträger (irregulärer Bruch) wurde mit einer gemäß dem Beispielkatalysator 1 der DE-A 2235103 präparierten, salzsauren Vanadium/Titan-Lösung im Vakuum imprägniert und entsprechend diesem Beispiel getrocknet und calciniert.
134.5 g des auf 4 mm gesiebten Bruches wurden in den Reaktor (Füllhöhe 1400 mm) gefüllt. Bei einer Salzbadtemperatur von 200°C wurde ein Butenumsatz von 96 % erreicht, die Essigsäureausbeute betrug nur 94 %. Dieser Katalysator liefert deutlich mehr Nebenprodukte (Maleinsäure, Propionsäure).

TABELLE 1:

| Bsp. | Katalysator | Reaktionsbedingungen | Temperatur [°C] | Umsatz [%] | Ausbeute [%] |
|------|-------------|---------------------|-----------------|------------|--------------|
| 1    | 2-Schalen   | Standard            | 213.5           | 88.6       | 116.25       |

TABELLE 1: (fortgesetzt)

| Bsp. | Katalysator | Reaktionsbedingungen | Temperatur [°C] | Umsatz [%] | Ausbeute [%] |
|------|-------------|---------------------|-----------------|------------|--------------|
| 2 | wenig Oberfläche | Standard | 198 | 88.0 | 116.5 |
| 3 | wenig Oberfläche | viel Dampf | 197 | 75.0 | 103.0 |
| 4 | dicke Schicht | viel Dampf | 196 | 95.0 | 113.5 |
| 5 | hohe V-Menge | viel Dampf | 189 | 92.0 | 105.0 |
| 6 | W-Dotierung | Standard | 179 | 95.0 | 116.2 |
| 7 | wenig C$_4$ | wenig Buten | 192.5 | 96.0 | 128.1 |
| 8 | hohe Oberfläche | Standard | 174 | 96.2 | 133.0 |
| 9 | Cs/P-Dotierung | Standard | 221.5 | 94.0 | 105.5 |
| 10 | Mo-Dotierung | Standard | 194 | 93.0 | 112.4 |
| 11 | 2-Schalen | Standard | 185 | 92.0 | 116.0 |
| 12 | Mo/Na-Dotierung | Standard | 252 | 92.0 | 96.4 |
| 13 | wenig Vanadium | Standard | 179 | 95.0 | 121.3 |
| 14 | S-Dotierung | Standard | 172.5 | 95.0 | 130.0 |
| 15 | hoher Mo-Gehalt | Standard | 183.5 | 94.5 | 120.0 |
| 16 | Sb-Dotierung | Standard | 175 | 94.4 | 142.0 |
| 17 | Bi-Dotierung | Standard | 179 | 90.2 | 120.0 |
| 18 | Te-Dotierung | Standard | 203 | 88.0 | 103.0 |
| 19 | Mn-Dotierung | Standard | 194 | 90.0 | 111.3 |
| 20 | Cu-Dotierung | Standard | 180 | 94.0 | 120.0 |
| 21 | Zn-Dotierung | Standard | 178.5 | 94.6 | 124.2 |
| 22 | Au-Dotierung | Standard | 182 | 95.8 | 128.4 |
| 23 | Cr-Dotierung | Standard | 184 | 95.0 | 128.0 |
| 24 | Ti/Sn-Katalysator | Standard | 191 | 94.0 | 130.0 |
| 25 | Ti/Zr-Katalysator | Standard | 201 | 93.0 | 112.0 |
| 26 | Ti/Zr-Katalysator | Standard | 199 | 95.0 | 115.0 |
| 27 | Nb-Dotierung | Standard | 219 | 96.0 | 105.0 |
| V1 | Vollkontakt | Standard | 247 | 90.0 | 77.0 |
| V2 | DE-A 2235103 | Standard | 200 | 96.0 | 94.0 |

**Patentansprüche**

1. Verwendung eines Schalenkatalysators bestehend aus einem inerten unporösen Trägerkörper und einer auf die äußere Oberfläche des Trägerkörpers aufgebrachten katalytisch aktiven Mischoxidmasse, welche

   a) ein oder mehrere Oxide aus der Gruppe Titandioxid, Zirkoniumdioxid, Zinndioxid, Aluminiumoxid und
   b) 0.1 bis 1.5 Gew%, bezogen auf das Gewicht der Komponente a) und pro m$^2$/g spezifischer Oberfläche der Komponente a), Vanadiumpentoxid enthält, zur Herstellung von Essigsäure durch Gasphasenoxidation von ungesättigten C$_4$-Kohlenwasserstoffen.

2. Verwendung nach Anspruch 1, dadurch gekennzeichnet, daß als Komponente a) zusätzlich noch ein oder mehrere Oxide der Metalle aus der Gruppe Hafnium, Niob, Wolfram, Lanthan und Cer, in einer Menge von 1 bis 15 Gew%, bezogen auf das Gesamtgewicht der Komponente a), enthalten sind.

3. Verwendung nach Anspruch 1 oder 2, dadurch gekennzeichnet, daß als Komponente b) ein Teil des Vanadiumpentoxids durch ein oder mehrere Oxide von Molybdän, Chrom und Antimon ersetzt ist, und/oder als zusätzliche Komponente b) noch ein oder mehrere Oxide von Alkalimetallen, Elementen der 5. und 6. Hauptgruppe des Periodensystems der Elemente (PSE) und der Übergangsmetalle enthalten sind.

4. Verwendung nach Anspruch 1 bis 3, dadurch gekennzeichnet, daß die katalytisch aktive Mischoxidmasse in einem Anteil von 1 bis 40 Gew%, bezogen auf das Gesamtgewicht aus Trägerkörper und aktiver Masse, als Schale, mit einer Schichtdicke von 10 bis 1000 μm, auf die äußere Oberfläche des Trägerkörpers aufgebracht ist.

**5.** Verwendung nach Anspruch 1 bis 4, dadurch gekennzeichnet, daß der Schalenkatalysator ein oder mehrere Schichten der katalytisch aktiven Mischoxidmasse enthält.

**6.** Verwendung nach Anspruch 1 bis 5, dadurch gekennzeichnet, daß der Schalenkatalysator mehrere Schichten enthält, wobei die innere Schicht nur Komponente a) enthält und die äußere Schicht die Komponenten a) und b).

**7.** Verwendung nach Anspruch 1 bis 5, dadurch gekennzeichnet, daß der Schalenkatalysator mehrere Schichten enthält, wobei die innere und äußere Schicht jeweils die Komponenten a) und b) enthalten, und für die innere Schicht eine höhere spezifische Oberfläche für die Komponente a) gewählt wird, als für die äußere Schicht.

**8.** Verfahren zur Herstellung von Essigsäure durch Gasphasenoxidation von ungesättigten $C_4$-Kohlenwasserstoffen in einem Rohrreaktor unter Verwendung eines Schalenkatalysators gemäß Anspruch 1 bis 7, wobei ein Gasgemisch, enthaltend Sauerstoff oder sauerstoffhaltiges Gas, ein oder mehrere $C_4$-Kohlenwasserstoffe und Wasserdampf, mit einem Volumenmischungsverhältnis Buten (mischung)/Sauerstoff (Luft) von 0.2/9.9.5 bis 15/85 und einem Volumenmischungsverhältnis Buten(mischung)/Wasserdampf von 1/1 bis 1/50, bei einer Temperatur von 100°C bis 400°C, und einem Überdruck von 0.2 bis 30 bar an dem Schalenkatalysator umgesetzt wird.

**9.** Verfahren zur Herstellung von Essigsäure durch Gasphasenoxidation von ungesättigten $C_4$-Kohlenwasserstoffen in einem Rohrreaktor nach Anspruch 8, dadurch gekennzeichnet, daß der Schalenkatalysator gemäß Anspruch 1 bis 7 zonenweise in die Reaktionsrohre eingebracht wird.

**Claims**

**1.** Use of a coated catalyst which consists of an inert nonporous support body and a catalytically active mixed oxide composition which is applied to the outer surface of the support body and contains

> a) one or more oxides selected from the group consisting of titanium dioxide, zirconium dioxide, tin dioxide, aluminium oxide and
> b) from 0.1 to 1.5% by weight, based on the weight of the component a) and per $m^2/g$ of specific surface area of the component a), of vanadium pentoxide, for preparing acetic acid by gas-phase oxidation of unsaturated $C_4$-hydrocarbons.

**2.** Use according to Claim 1, characterized in that component a) additionally contains one or more oxides of the metals selected from the group consisting of hafnium, niobium, tungsten, lanthanum and cerium in an amount of from 1 to 15% by weight, based on the total weight of the component a).

**3.** Use according to Claim 1 or 2, characterized in that in component b) part of the vanadium pentoxide is replaced by one or more oxides of molybdenum, chromium and antimony, and/or one or more oxides of alkali metals, elements of main groups V and VI of the Periodic Table of the Elements and the transition metals are present as additional component b).

**4.** Use according to any of Claims 1 to 3, characterized in that the catalytically active mixed oxide composition is applied to the outer surface of the support body in an amount of from 1 to 40% by weight, based on the total weight of support body and active composition, as a shell having a layer thickness of from 10 to 1000 µm.

**5.** Use according to any of Claims 1 to 4, characterized in that the coated catalyst contains one or more layers of the catalytically active mixed oxide composition.

**6.** Use according to any of Claims 1 to 5, characterized in that the coated catalyst contains a plurality of layers, where the inner layer contains only component a) and the outer layer contains the components a) and b).

**7.** Use according to any of Claims 1 to 5, characterized in that the coated catalyst contains a plurality of layers, where the inner and outer layers each contain the components a) and b) and the specific surface area selected for the component a) is higher for the inner layer than for the outer layer.

**8.** Process for preparing acetic acid by gas-phase oxidation of unsaturated $C_4$-hydrocarbons in a tube reactor using a coated catalyst according to any of Claims 1 to 7, wherein a gas mixture containing oxygen or oxygen-containing gas, one or more $C_4$-hydrocarbons and water vapour and having a volume mixing ratio of butene (mixture)/oxygen (air) of from 0.2/99.5 to 15/85 and a volume mixing ratio of butene(mixture)/water vapour of from 1/1 to 1/50 is reacted over the coated catalyst at a temperature of from 100°C to 400°C and a gauge pressure of from 0.2 to 30 bar.

9. Process for preparing acetic acid by gas-phase oxidation of unsaturated $C_4$-hydrocarbons in a tube reactor according to Claim 8, characterized in that the coated catalyst according to any of Claims 1 to 7 is introduced in zones into the reaction tubes.


**Revendications**

1. Utilisation d'un catalyseur à coquille, constitué d'un corps support inerte non poreux et d'une masse d'oxydes mixtes catalytiquement active appliquée sur la surface extérieure du corps support, laquelle contient

   a) un ou plusieurs oxydes du groupe dioxyde de titane, dioxyde de zirconium, dioxyde d'étain, oxyde d'aluminium et
   b) 0,1 à 1,5% en poids, par rapport au poids du composant a) et par $m^2/g$ de surface spécifique du composant a), de pentoxyde de vanadium pour la production d'acide acétique par oxydation en phase gazeuse d'hydrocarbures insaturés en $C_4$.

2. Utilisation selon la revendication 1, caractérisée en ce que sont en outre encore contenus comme composant a), un ou plusieurs oxydes des métaux du groupe hafnium, niobium, tungstène, lanthane et cérium, en une quantité de 1 à 15% en poids par rapport au poids total du composant a).

3. Utilisation selon la revendication 1 ou 2, caractérisée en ce que, en tant que composant b), une partie du pentoxyde de vanadium est remplacée par un ou plusieurs oxydes de molybdène, de chrome et d'antimoine et/ou en tant que composant b) supplémentaire, encore un ou plusieurs oxydes de métaux alcalins, d'éléments du 5ème et 6ème groupe principal du système périodique des éléments (SPE) et des métaux de transition sont contenus.

4. Utilisation selon les revendications 1 à 3, caractérisée en ce que la masse d'oxydes mixtes catalytiquement active est appliquée, en une proportion de 1 à 40% en poids, par rapport au poids total de corps support et de masse active, en tant que coquille présentant une épaisseur de couche de 10 à 1000 μm, sur la surface extérieure du corps support.

5. Utilisation selon les revendications 1 à 4, caractérisée en ce que le catalyseur à coquille contient une ou plusieurs couches de la masse d'oxydes mixtes catalytiquement active.

6. Utilisation selon les revendications 1 à 5, caractérisée en ce que le catalyseur à coquille contient plusieurs couches, la couche interne ne contenant que le composant a) et la couche externe les composants a) et b).

7. Utilisation selon les revendications 1 à 5, caractérisée en ce que le catalyseur à coquille contient plusieurs couches, la couche interne et la couche externe contenant à chaque fois les composants a) et b) et une surface spécifique plus grande pour le composant a) étant choisie pour la couche interne que pour la couche externe.

8. Procédé pour la production d'acide acétique par oxydation en phase gazeuse d'hydrocarbures insaturés en $C_4$ dans un réacteur tubulaire avec utilisation d'un catalyseur à coquille selon les revendications 1 à 7, un mélange gazeux, contenant de l'oxygène ou un gaz contenant de l'oxygène, un ou plusieurs hydrocarbures en $C_4$ et de la vapeur d'eau, présentant un rapport de mélange volumique butène (mélange)/oxygène (air) de 0,2/99,5 à 15/85 et un rapport de mélange volumique butène (mélange)/vapeur d'eau de 1/1 à 1/50, étant transformés à une température de 100°C à 400°C et à une surpression de 0,2 à 30 bars sur le catalyseur à coquille.

9. Procédé pour la production d'acide acétique par oxydation en phase gazeuse d'hydrocarbures insaturés en $C_4$ dans un réacteur tubulaire selon la revendication 8, caractérisé en ce que le catalyseur à coquille selon la revendication 1 à 7 est introduit en zones dans les tubes de réaction.